# EUROPEAN PATENT APPLICATION

(11) **EP 4 652 979 A2**
(43) Date of publication of application: **26.11.2025**
(21) Application number: 25178631.5
(22) Date of filing: 23.05.2025
(51) Int. Cl.: A61H 15/00, A63F 9/34, A63H 33/00

(54) **MANIPULATIVE DEVICE**

(30) Priority: 24.05.2024 US 202463651880 P; 21.03.2025 US 202519087184
(71) Applicant: Horowitz, Ari, Brooklyn, NY 11249 (US)
(72) Inventor: Horowitz, Ari, Brooklyn, NY 11249 (US)
(74) Representative: Soldatini, Andrea

(57) **Abstract**

A manipulative device having at least two three-dimensional bodies, each including a magnet. The at least two three-dimensional bodies including at least one first three-dimensional body and at least one second three-dimensional body. The at least one first three-dimensional body is manipulated by a user relative to the at least one second three-dimensional body, whereby the surface of the at least first three-dimensional body contacts the surface of the at least second three-dimensional body, and the at least two three-dimensional bodies engage or repel each other due to magnetic force from the magnet in each three-dimensional body. Also, the at least two three-dimensional bodies disengage or release from each other due to reduction in magnetic force from the magnet in each three-dimensional body disconnecting when separating the three-dimensional bodies from each other. A sound or noise dampening material can cover the entire surface of each three-dimensional body.

## Description

### BACKGROUND OF THE INVENTION

The background of the invention is in manipulative devices, fidget devices, concentration trainers, and dexterity trainers. Devices such as the present invention can help with stress relief, exercise, diversion, focus, dexterity, etc.

### SUMMARY OF THE INVENTION

In one embodiment, the invention is a manipulative device having at least two three-dimensional bodies. Each three-dimensional body has a surface and includes a magnet. The at least two three-dimensional bodies including at least one first three-dimensional body and at least one second three-dimensional body. The at least one first three-dimensional body is manipulated by a user relative to the at least one second three-dimensional body, whereby the surface of the at least first three-dimensional body contacts the surface of the at least second three-dimensional body, and the at least two three-dimensional bodies hold on to or engage each other due to magnetic force from the magnet in each three-dimensional body. Also, the at least two three-dimensional bodies disengage or release from each other due to reduction in magnetic force from the magnet in each three-dimensional body disconnecting when separating the three-dimensional bodies from each other. A sound or noise dampening material covers the surface of each three-dimensional body, whereby sound is decreased where the three-dimensional bodies connect or come into contact. The sound or noise dampening material can cover the entire surface of each three-dimensional body for optimum sound or noise dampening.

In another embodiment, the sound dampening material covering the surface of each three-dimensional body is a flexible or soft material.

In another embodiment, the flexible or soft material is silicone.

In another embodiment, the silicone is an anti-static silicone. The anti-static silicone should be able to stay cleaner.

In another embodiment, the sound dampening material covering the surface of each three-dimensional body is silicone, flexible or soft rubber, flexible or soft plastic, flexible or soft wood or a combination of two or more of these materials. Hard plastic is known for covering magnetic manipulative devices and the result is noisy when the devices come into contact with each other or other surfaces.

In another embodiment, the three-dimensional bodies are shaped like different sports balls, for example, golf balls, footballs, foosballs, baseballs, basketballs, rugby balls, soccer balls, volleyballs, etc. Additionally, the three-dimensional bodies can have different textures on their surfaces. For example, the three-dimensional bodies can be textured like the afore-mentioned sports balls, they can have nubby protrusions, dimples, ribs, spiky protrusions, etc.

The three-dimensional bodies can be various shapes that can be manipulated as a manipulative device. The different three-dimensional bodies do not have to be the same shape and can be different shapes and textures. In one embodiment, the three-dimensional bodies are both (all) spheres.

The three-dimensional bodies can be various shapes that can be manipulated as a manipulative device. The different three-dimensional bodies do not have to be the same shape and can be different shapes. In one embodiment, one or more of the three-dimensional bodies are three dimensional shapes, for example, cones, cubes, cuboids, ellipsoids, icosahedrons, prisms, nonahedrons, heptagonal prisms, octagonal prisms, nonagonal prisms, pyramids, octahedrons, pentahedrons, pentagonal prisms, heptahedrons, hexahedrons, cylinders, triangular prisms, or irregular shapes. Irregular shapes can include free-form shapes created from the imagination, like a work of art, shapes produced as a product or a by-product of a natural or synthetic process (e.g., digestion, protrusion, etc.), shapes created by nature (e.g., a carrot, pinecone, penguin, etc.), etc.

In one embodiment, the size of each three-dimensional body has a length to width to depth ratio of about 1:1:1, 1:1:3, 1:2:2.

The manipulative device of the present invention includes at least two three-dimensional bodies to manipulate. However, the inventive manipulative device can include as many three-dimensional devices as desired. Up to eight three-dimensional bodies are described herein, but more can be included if desired.

One embodiment of the manipulative device further includes at least one third three-dimensional body.

Another embodiment of the manipulative device further includes at least one fourth three-dimensional body.

Another embodiment of the manipulative device further includes at least one fifth three-dimensional body.

Another embodiment of the manipulative device further includes at least one sixth three-dimensional body.

Another embodiment of the manipulative device further includes at least one seventh three-dimensional body.

Another embodiment of the manipulative device further includes at least one eighth three-dimensional body.

In one embodiment, the manipulative device can be used by connecting the at least two three-dimensional bodies together to form a chain of the at least two three-dimensional bodies. In one embodiment, the chain can be a magnetic chain.

In further embodiments, the repelling function of magnets can also be employed. By way of non-limiting example, the chain of at least two three-dimensional bodies can be used together with one or more additional other three-dimensional bodies which repel the chain and cause it to move around in the opposite direction. (When the attracting function of magnets is used, it can cause the chain to move in the same direction.) The one or more additional three-dimensional bodies can act on the chain when the chain is held in the air and the one or more additional three-dimensional bodies is positioned close enough to repel or attract the chain and make it move, or when the chain is lying against a surface and the one or more additional three-dimensional bodies is positioned close enough to repel or attract the chain and make it move (in this example, the one or more additional three-dimensional bodies can be positioned on the surface too, above the surface, below the surface, etc.

In a further embodiment, the chain is dragged around (e.g., in the air on the surface of another object, e.g., a table).

A further embodiment includes removing (popping) at least one of the at least two three-dimensional bodies off the chain, whereby the removed body stays within the magnetic force of the body or bodies remaining in the chain, the removed body hovers, and is pulled back by the magnetic force of one or more of the at least two three-dimensional bodies remaining in the chain.

A further embodiment includes substituting (replacing) one of the at least two three-dimensional bodies for another of the at least two three-dimensional bodies in the chain.

In another embodiment, the manipulative device can be used by connecting the at least two three-dimensional bodies together to form a chain of the at least two three-dimensional bodies, and connecting them to build them into a shape. They can form various shapes when connected.

In another embodiment, the manipulative device can be used by connecting the at least two three-dimensional bodies together to form a chain of the at least two three-dimensional bodies, and connecting the three-dimensional bodies at each end of the chain to form a circle shape.

In another embodiment, the manipulative device can be used by revolving the three-dimensional bodies around each other while rolling them around in a user's hand or by a user's fingers, which causes contact between the three-dimensional bodies, and the contact results in magnetic attraction and connection between the three-dimensional bodies. Then, continuing to roll the three-dimensional bodies around in the user's hand or by a user's finger's causes the three-dimensional bodies to separate, for example, the three-dimensional bodies can be separated by a user's fingers, thereby breaking the magnetic attraction/field, and allowing the three-dimensional bodies to disconnect.

In a further embodiment, the magnetic force is strong enough to hold the three-dimensional bodies together, but weak enough so that the three-dimensional bodies can be separated for use as described.

In another embodiment, the manipulative device has a storage container comprising a bottom tray, a top lid, which covers the bottom tray, at least one magnet in the top lid matching up to at least one magnet in the bottom tray, when the top lid covers the bottom tray, and the bottom tray having an indented region below its at least one magnet. The indented region fits the at least one magnet in the overturned top lid, whereby the bottom tray nests inside the top lid.

In one embodiment, the bottom tray has a first end and a second end. The top cover also has a first end and a second end, each of which, respectfully and interchangeably, match up with the first end and the second end of the bottom tray when the top lid covers the bottom tray. There can be a first magnet in the first end or the second end of the bottom tray and a second magnet in the first end or the second end of the top lid. Also, the bottom tray has an indented region below the first magnet in the first end or the second end to fit the second magnet in the first end or the second end of the top lid, whereby the bottom tray nests inside the top cover.

In another embodiment, the storage container also includes a third magnet in the bottom tray, whereby the first magnet is in the first end of the bottom tray and the third magnet is in the second end of the bottom tray. Additionally, the storage container also includes a fourth magnet in the top lid, whereby the second magnet is in the first end of the bottom tray and the fourth magnet is in the second end of the top lid.

In another embodiment, the storage container also includes a ridge extending substantially between the first end and the second end of the bottom tray or the top lid. The ridge can be used to keep the three-dimensional bodies in place in the storage container, The ridge does not have to extend the entire length of the bottom tray or the top lid, but can extend part of the length between the first end and the second end of either or both of the bottom tray or the top lid, a length sufficient to hold one or more three-dimensional bodies in place. Also, the ridge can extend the entire length of the bottom tray or the top lid, if desired.

In another embodiment of the storage container, the top lid also includes an opening notch and the bottom tray has an opening notch which matches up with the opening notch in the top lid when the top lid covers the bottom tray. A user can use the opening notch to separate the top lid from the bottom tray and open the storage container.

In one embodiment, the invention is a manipulative device having at least two three-dimensional bodies, each three-dimensional body having a surface. At least one of the three-dimensional bodies including a magnet and the other of the three-dimensional bodies including a magnet or being made of a ferromagnetic material. The three-dimensional body(ies), which include a magnet, having a surface fully encased in a sound dampening material, to decrease sound where the at least two three-dimensional bodies connect. The at least two three-dimensional bodies forming a chain (as described above) and/or revolving around each other, whereby the surface of a three-dimensional body connects to another three-dimensional body due to magnetic force and, then disconnects from the other three-dimensional body due to separating the three-dimensional bodies from each other.

In one embodiment, the invention is used by revolving the three-dimensional bodies around each other while rolling them around in a user's hand or by a user's fingers, which causes contact between the three-dimensional bodies, and the contact results in magnetic attraction and connection between the three-dimensional bodies. Then, continuing to roll the three-dimensional bodies around in the user's hand or by a user's fingers causes the three-dimensional bodies to separate (for example, the fingers can separate the three-dimensional bodies), thereby breaking the magnetic attraction/field, and allowing the three-dimensional bodies to disconnect. The inventive device can be held in a user's palm, and contained within a user's palm and finger digits, while the user can roll/rotate one or more of the three-dimensional bodies. The user can roll/rotate one or more of the three-dimensional bodies using one or more digits (of a user).

### BRIEF DESCRIPTION OF DRAWINGS

Fig. 1 is a front elevation view of one embodiment of the invention;
Fig. 2 is a top perspective view of another embodiment of the invention;
Fig. 3 is a top view of another embodiment of the invention;
Fig. 4 is a top perspective view of one embodiment of a storage case for three-dimensional bodies of the inventive Manipulative Device;
Fig. 5 is a top perspective view of the bottom piece of the storage case nestled within the overturned top piece of the storage case, for ease of illustration;
Fig. 6 is a top perspective view of the top of the storage case floating above the bottom of the storage case filled with three-dimensional bodies, for ease of illustration;
Fig. 7 is a top perspective view of the top of the storage case overturned and floating above the bottom of the storage case filled with three-dimensional bodies, for ease of illustration;
Fig. 8 is another embodiment of a three-dimensional body;
Fig. 9 is an example of a stack of four three-dimensional bodies of the embodiment shown in Fig. 8;
Fig. 10 is an example of a stack of three three-dimensional bodies of the embodiment shown in Fig. 8;

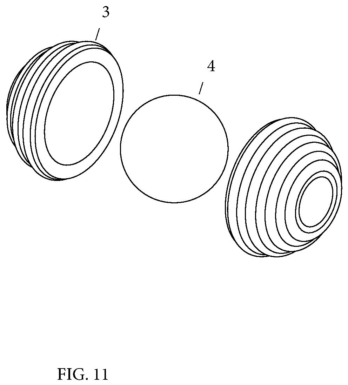

Fig. 11 is an exploded view showing one embodiment of a magnet inside a three-dimensional body; and

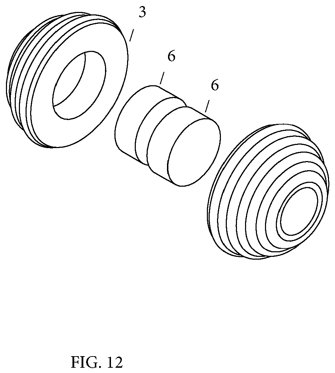

Fig. 12 is an exploded view showing one embodiment of multiple magnets inside a three-dimensional body.

### DETAILED DESCRIPTION OF THE INVENTION

### Key to Figures:

1 is one embodiment of a manipulative device according to the invention;
2 is one embodiment of a three-dimensional body (this non-limiting example exhibits a spherically shaped body);
3 is a surface of a three-dimensional body covered by a sound dampening material;
4 is one embodiment of a single magnet for a three-dimensional body;
6 is one embodiment of multiple magnets for a three-dimensional body;
5 is a nubby protrusion;
7 is a dimple;
9 is a spiky protrusion;
11 is a rib (this rib 11 is shown as part of a set of concentric ribs, by way of a non-limiting example on how ribs can be arranged on a three-dimensional body);
13 is a chain of three-dimensional bodies;
15 is a chain of three-dimensional bodies forming a circular shape;
17 is one example of a storage container for three-dimensional bodies 2 of the inventive Manipulative Device 1;
19 is a top lid of the storage case 17;
21 is a bottom tray of the storage case 17;
23 is a notch in the bottom 21 of the storage case 17;
25 is an opening in the storage case 17 created by a matching indent in both the top 19 and bottom 21 of the storage case 17;
27 is one of two magnets in the bottom 21 of the storage case;
29 is a ridge on the inside of the bottom 21 of the storage case 17, which can be used to hold the three-dimensional bodies 2 in place in the storage case 17, it can be used as a dividing ridge;
31 is one of two magnets in the top 19 of the storage case 17;
32 is another embodiment of a manipulative device according to the invention;
33 is another embodiment of a three-dimensional body (this non-limiting example exhibits a cuboid shaped body).

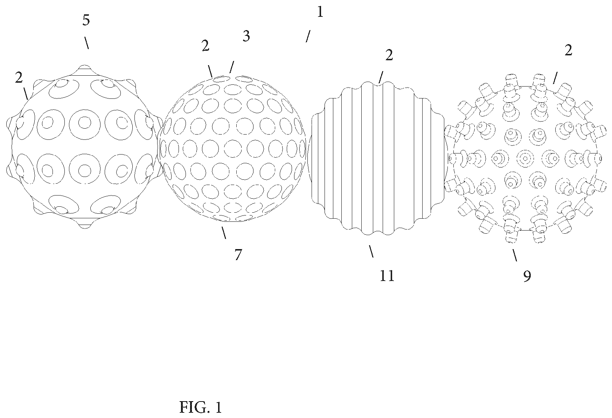

Fig. 1 shows an embodiment of the invention including one embodiment of the inventive manipulative device 1 with four three-dimensional bodies 2, all of them being spheres and having textured surfaces 3. The first one having regularly spaced nubby protrusions 5, the second one having golf ball-type dimples 7, the third one having circular concentric ribs 11 and the fourth one having irregularly spaced spiky protrusions 9.

The manipulative device 1 of the present invention can be made from as few as two three-dimensional bodies 2. The manipulative device 1 has at least two three-dimensional bodies 2, each three-dimensional body 2 has a surface 3 and includes a magnet. Although, each three-dimensional body 2 can include more than one magnet.

The at least two three-dimensional bodies 2 include at least one first three-dimensional body 2 and at least one second three-dimensional body 2. The at least one first three-dimensional body 2 can be manipulated by a user relative to the at least one second three-dimensional body 2, whereby the surface of the at least first three-dimensional body 2 contacts the surface of the at least second three-dimensional body 2, and the at least two three-dimensional bodies 2 hold on to (or engage) each other due to magnetic force from the magnet(s) in each three-dimensional body 2, and the at least two three-dimensional bodies 2 disengage from each other due to reduction in magnetic force from the magnet in each three-dimensional body 2 disconnecting when separating the three-dimensional bodies 2 from each other.

A sound or noise dampening material 3 can cover the surface of each three-dimensional body 2, to decrease any sound or noise where the three-dimensional bodies 2 connect or come into contact. The sound dampening material 3 can cover the entire surface of the three-dimensional body 2 to ensure a more complete sound-dampening effect. In an alternative embodiment of the manipulative device, the surface of at least one of the three-dimensional bodies 2 is covered by the sound dampening material 3, but another of the three-dimensional bodies 2 is a magnet without a sound dampening material 3 on its surface. In that way, quiet manipulation of the inventive manipulative device 1 can be pursued using a three-dimensional body 2 without a sound dampening material 3 together with a three-dimensional body having a sound dampening material 3.

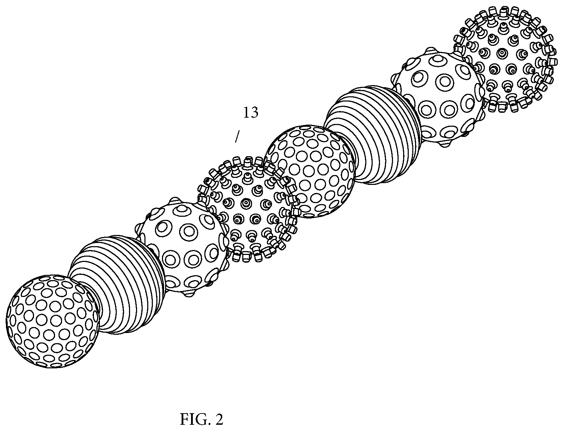

Fig. 2 shows another embodiment of the invention including eight three-dimensional bodies 2 in a chain 13, all of them being spheres and having the same textured surfaces as in the embodiment of Fig. 1. In both the embodiments of Figs. 1 and 2 the three-dimensional bodies 2 are placed end-to-end to form a chain 13. This chain 13 can be a magnetic chain because the three-dimensional bodies 2 include magnets and the chain 13 can be held together by magnetic force. In one embodiment, each of the three-dimensional bodies 2 includes a single magnet. However, each of the three-dimensional bodies 2 can include more than one magnet.

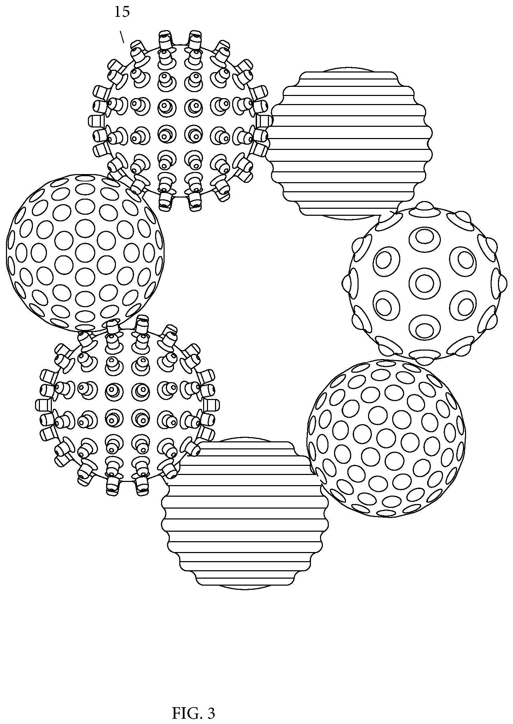

The chain 13 formed by the three-dimensional bodies can be made from as few as two three-dimensional bodies 2. Once the three-dimensional bodies 2 are connected as a chain 13, a user can drag the chain 13 around in the air, or on any type of surface. Additionally, the chain 13 can be formed into a shape, such as a circle 15, as shown in Fig. 3. Also, two or more chains 13 and/or circles 15 and/or three-dimensional bodies 2 can be joined together to build a larger manipulative device 1 from the three-dimensional bodies 2.

Further, when the inventive manipulative device 1 is in the form of a chain 13 of at least two three-dimensional bodies 2, a user can manipulate it by slightly removing at least one of the at least two three-dimensional bodies 2 off the chain 13. When the at least one of the at least two three-dimensional bodies 2 is only slightly removed from off the chain 13, like it's being popped off, it can stay within the magnetic force between the three-dimensional body 2 being removed and any one or more three-dimensional body(ies) 2 remaining on the chain 13. Because the three-dimensional body 2 being slightly removed remains within that magnetic force, it hovers, and is pulled back by another of the at least two three-dimensional bodies remaining in the chain 13. This type of manipulation of manipulative device 1 can be carried out in various ways, by way of non-limiting example, by a user holding the chain 13 in the user's hand and using the user's thumb and/or finger(s) to slightly separate one or more three-dimensional bodies 2 from the chain 13, let them hover and reattach to the chain 13, or by a user holding the chain 13 on a surface and using the user's thumb and/or finger(s) to slightly separate one or more three-dimensional bodies 2 from the chain 13, let them separate and reattach to the chain 13, etc.

Still further, when the inventive manipulative device 1 is in the form of a chain 13 of at least two three-dimensional bodies 2, a user can manipulate it by substituting (or replacing) one of the at least two three-dimensional bodies 2 for another of the at least two three-dimensional bodies 2 in the chain 13.

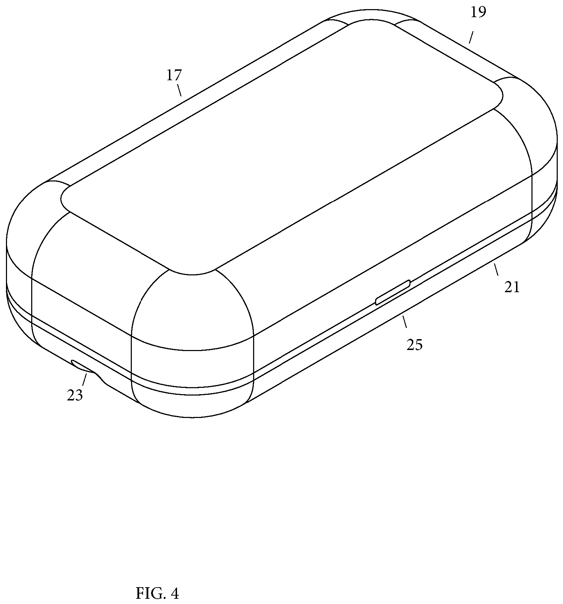

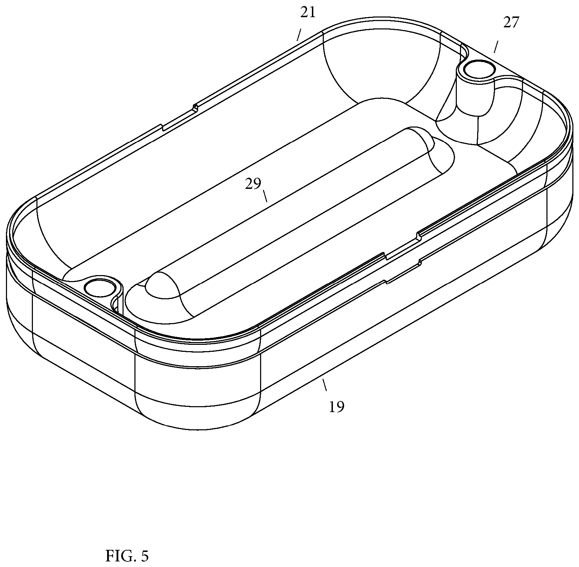

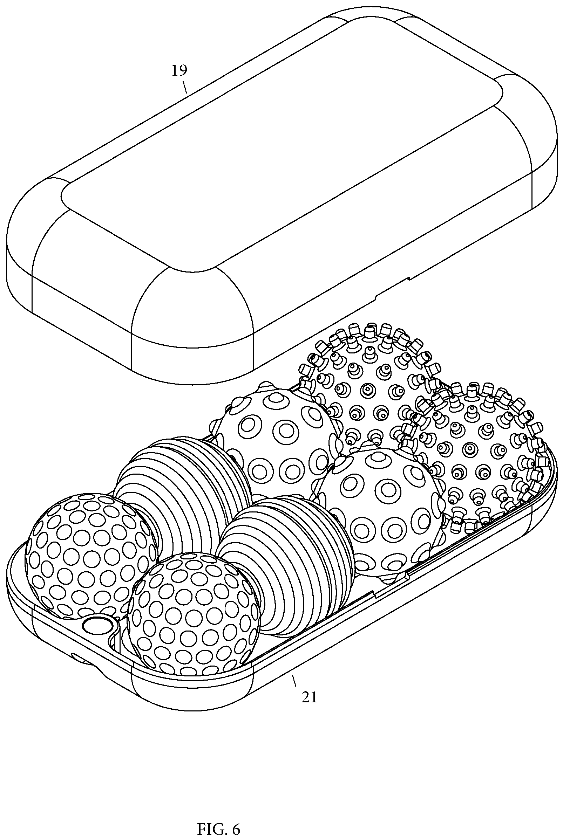

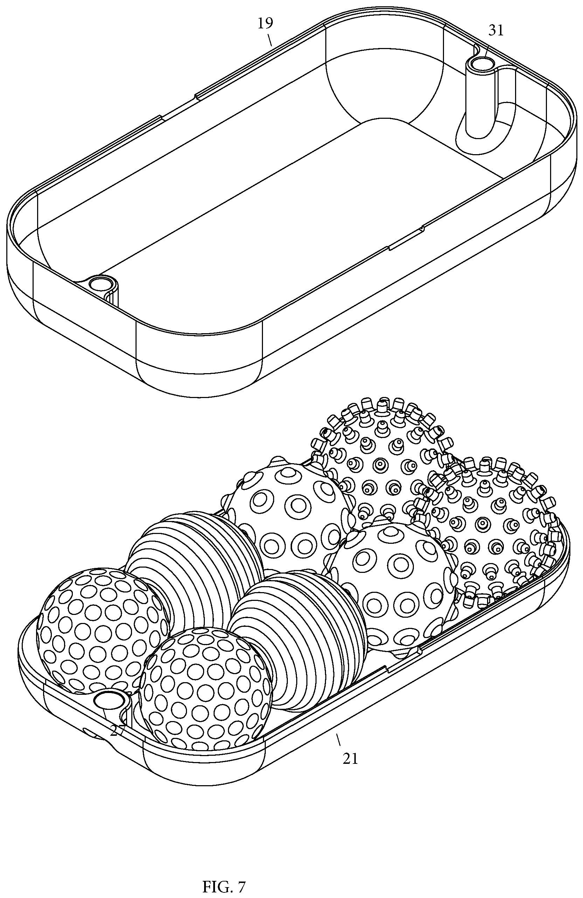

In another embodiment shown in Figs. 4-7, the manipulative device 1 has a storage container 17 comprising a bottom tray 21, a top lid 19, which covers the bottom tray 21, at least one magnet 31 in the top lid 19 matching up to at least one magnet 27 in the bottom tray 21, when the top lid 19 covers the bottom tray 21, also the bottom tray 21 has an indented region below its at least one magnet. The indented region fits the at least one magnet in the overturned top lid 19, whereby the bottom tray 21 can nest inside the top lid 19.

In one embodiment, the bottom tray 21 has a first end and a second end. The top cover 19 also has a first end and a second end, each of which, respectfully and interchangeably, match up with the first end and the second end of the bottom tray 21 when the top lid 19 covers the bottom tray 21. There can be a first magnet in the first end or the second end of the bottom tray 21 and a second magnet in the first end or the second end of the top lid 19, and these magnets can connect when the top lid 19 covers the bottom tray 21. Also, the bottom tray 21 has an indented region 23 below the first magnet in the first end or the second end to fit the second magnet in the first end or the second end of the top lid 19, whereby the bottom tray 21 can nest inside the top cover 19.

In another embodiment, the storage container 17 also includes a third magnet 27 in the bottom tray 21, whereby the first magnet 27 is in the first end of the bottom tray 21 and the third magnet 27 is in the second end of the bottom tray 21, and a fourth magnet 31 in the top lid 19, whereby the second magnet 31 is in the first end of the top lid 19 and the fourth magnet 31 is in the second end of the top lid 19.

In another embodiment, the storage container 17 also includes a ridge or divider 29 extending substantially between the first end and the second end of the bottom tray 21 or the top lid 19. The ridge 29 can be used to keep the three-dimensional bodies 2 in place in the storage container 17, The ridge 29 does not have to extend the entire length of the bottom tray 21 or the top lid 19, but can extend part of the length (or the entire length) between the first end and the second end of either or both of the bottom tray 21 or the top lid 19. The length ridge 29 extends should be sufficient to hold one or more three-dimensional bodies 2 in place.

In another embodiment of the storage container 17, the top lid 19 also includes an opening notch 25 and the bottom tray 21 has an opening notch 25 which matches up with the opening notch 25 in the top lid 19 when the top lid 19 covers the bottom tray 21. A user can use the opening notch 25 to separate the top lid 19 from the bottom tray 21 and open the storage container 17.

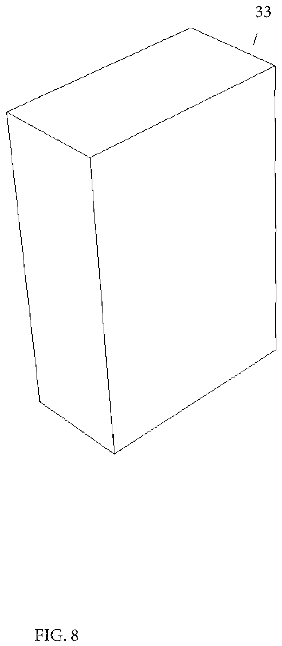

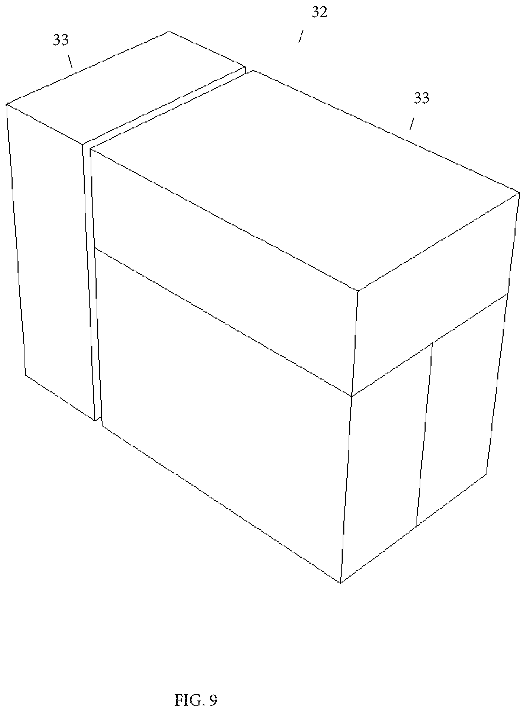

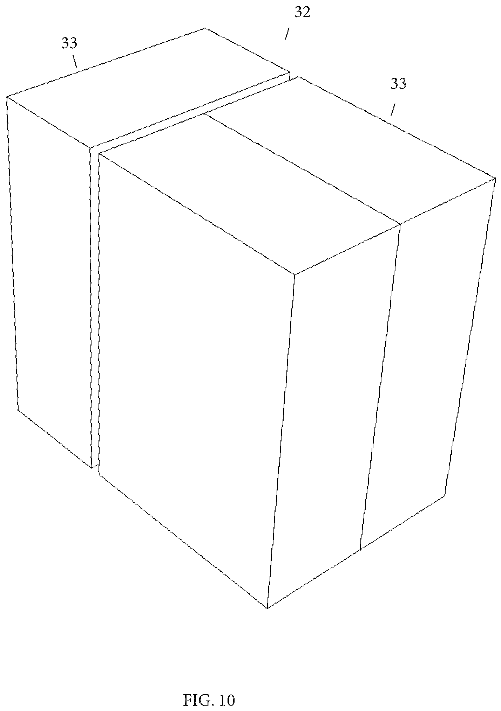

An exemplary alternative embodiment of a three-dimensional body 33 having a cuboid shape is shown in Fig. 8. Fig. 9 is an example of a stack of four three-dimensional bodies 33 of the embodiment shown in Fig. 8. In Fig. 9, another embodiment of the inventive manipulative device 32 is stacked to show that the three-dimensional bodies 33 can be used for building structures. Fig. 10 shows an embodiment of the manipulative device 32 of Fig. 9 with three three-dimensional bodies 33.

The three-dimensional bodies can be shaped like different sports balls, for example, golf balls, footballs, foosballs, baseballs, basketballs, rugby balls, soccer balls, volleyballs, etc. The three-dimensional bodies can also have the same patterns and textures as such sports balls. Additionally, or alternatively, the three-dimensional bodies can have various textures, such as bumps, protrusions, spikes and/or ribbing, etc. The three-dimensional bodies do not have to be uniform in size or shape in order to be used together. One example of a ferromagnetic object is a steel object. In one example, the magnet in the three-dimensional body or bodies is a single, solid magnet. In another example, the magnet is comprised of two or more magnets in the three-dimensional body or bodies. The three-dimensional bodies are sized to be held and manipulated within a user's hand, for example, about 0.5 inches to about 3.5 inches. The surface of the three-dimensional body or bodies are encased to the extent that sound can be avoided when the bodies connect with each other and/or when they connect with a three-dimensional body made of a ferromagnetic material. Encasing the magnet also has the benefits of rust proofing, and, where water can't get to it, neither can bacteria, so it's more hygienic. Hence, the greater extent to which the magnet(s) is encased, i.e., the surface 3 of the three-dimensional body 2 is covered, the greater extent of sound proofing, rust proofing, bacteria proofing, etc., with a completely covered surface 3 being optimally effective for such factors. Although the three-dimensional bodies, being used together in one set as a manipulative device, do not have to be the same size or shape, they also can be the same size or shape, and can even be multiples of each other as shown in the figures of the cuboid shapes.

One example of the size of each three-dimensional body is a length to width to depth ratio of about 2 to about 3 inches long, to about 1 inch wide to about 1 inch deep.

The manipulative device can be used by revolving the three-dimensional bodies around each other while rolling them around in a user's hand or by a user's fingers, which causes contact between the three-dimensional bodies, and the contact results in magnetic attraction and connection between the three-dimensional bodies. Then, continuing to roll the three-dimensional bodies around in the user's hand or by a user's fingers causes the three-dimensional bodies to separate (for example, the user's fingers can separate the three-dimensional bodies), thereby breaking the magnetic attraction/field, and allowing the three-dimensional bodies to disconnect.

Although certain embodiments of the present invention have been described it will be understood by those skilled in the art that the present invention should not be limited to the described embodiments. Rather, various changes and modifications can be made withinthe spirit and scope of the present invention as part thereof.

## Claims

1. A manipulative device comprising:
at least two three-dimensional bodies, each three-dimensional body having a surface;
each three-dimensional body including a magnet:
the at least two three-dimensional bodies including at least one first three-dimensional body and at least one second three-dimensional body;
the at least one first three-dimensional body being manipulated by a user relative to the at least one second three-dimensional body, whereby the surface of the at least first three-dimensional body contacts the surface of the at least second three-dimensional body, and the at least two three-dimensional bodies hold on to each other due to magnetic force from the magnet in each three-dimensional body, and the at least two three-dimensional bodies disengage from each other due to reduction in magnetic force from the magnet in each three-dimensional body disconnecting when separating the three-dimensional bodies from each other;
a sound dampening material covering the surface of each three-dimensional body, whereby sound is decreased where the three-dimensional bodies connect.

2. The manipulative device of claim 1, wherein the sound dampening material completely covers the surface of each three-dimensional body.

3. The manipulative device of claim 1, wherein the sound dampening material covering the surface of each three-dimensional body is a flexible material.

4. The manipulative device of claim 1, wherein the sound dampening material covering the surface of each three-dimensional body is silicone, flexible rubber, flexible plastic, flexible wood or a combination of two or more of these materials.

5. The manipulative device of claim 1, wherein the three-dimensional bodies are shaped like different sports balls, for example, golf balls, footballs, foosballs, baseballs, basketballs, rugby balls, soccer balls, or volleyballs.

6. The manipulative device of claim 1, wherein the three-dimensional bodies are spheres.

7. The manipulative device of claim 1, wherein the first three-dimensional body and the second three-dimensional body are three dimensional shapes, for example, cones, cubes, cuboids, ellipsoids, icosahedrons, prisms, nonahedrons, heptagonal prisms, octagonal prisms, nonagonal prisms, pyramids, octahedrons, pentahedrons, pentagonal prisms, heptahedrons, hexahedrons, cylinders, triangular prisms or irregular shapes.

8. The manipulative device of claim 1, wherein the manipulative device is used by connecting the at least two three-dimensional bodies together to form a chain of the at least two three-dimensional bodies.

9. The manipulative device of claim 8, further comprising removing at least one of the at least two three-dimensional bodies off the chain, whereby it stays within the magnetic force, hovers, and is pulled back by another of the at least two three-dimensional bodies remaining in the chain.

10. The manipulative device of claim 9, wherein the manipulative device is used by connecting the at least two three-dimensional bodies together to form a chain of the at least two three-dimensional bodies, and connecting them end to end.

11. The manipulative device of claim 1, wherein the manipulative device is used by revolving the three-dimensional bodies around each other while rolling them around in a user's hand or by a user's fingers, which causes contact between the three-dimensional bodies, and the contact results in magnetic attraction and connection between the three-dimensional bodies.

12. A storage container for the manipulative device of claim 1, the storage container comprising:
a bottom tray;
a top lid, which covers the bottom tray;
the bottom tray having a first end and a second end;
the top cover having a first end and a second end, each of which, respectfully and interchangeably, match up with the first end and the second end of the bottom tray when the top lid covers the bottom tray;
a first magnet in the first end or the second end of the bottom tray;
a second magnet in the first end or the second end of the top lid; and
the bottom tray having an indented region below the first magnet in the first end or the second end to fit the second magnet in the first end or the second end of the top lid, whereby the bottom tray nests inside the top cover.

13. The storage container of claim 12, further comprising:
a third magnet in the bottom tray, whereby the first magnet is in the first end of the bottom tray and the third magnet is in the second end of the bottom tray; and
a fourth magnet in the top lid, whereby the second magnet is in the first end of the bottom tray and the fourth magnet is in the second end of the top lid.

14. The storage container of claim 12, further comprising:
A ridge extending substantially between the first end and the second end of the bottom tray or the top lid.
